# EUROPÄISCHE PATENTANMELDUNG

(11) **EP 1 520 849 A1**
(43) Veröffentlichungstag der Anmeldung: **06.04.2005**
(21) Anmeldenummer: 04103410.9
(22) Anmeldetag: 16.07.2004
(51) Int. Cl.: C07C 213/06, C07C 215/08

(54) **Verfahren zur Herstellung von 2-(N,N-Dialkylamino)-Ethoxyverbindungen**

(30) Priorität: 16.09.2003 DE 10342647
(71) Anmelder: Degussa AG, 40474 Düsseldorf (DE)
(72) Erfinder: Bodmann, Kerstin Dr., 457752 Marl (DE); Imig, Manuela, 45768 Marl (DE)

(57) **Zusammenfassung**

Beschrieben wird ein Verfahren zur Herstellung von 2-(N,N-Dialkylamino)-ethoxyverbindunge der Formel I, 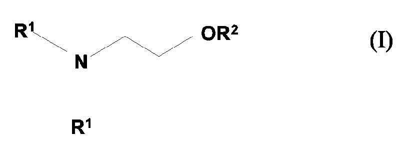 bei dem man 2- N,N-dialkylaminoethanol der Formel II 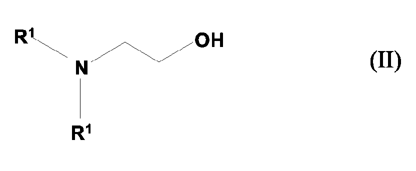 mit Alkylchloriden oder Alkoxyethylchloriden der Formel III

**CI**―**R**^{**2**} **(III)** (III)

in Gegenwart von wässrigen Alkalimetallhydroxidlösungen und Phasentransferkatalysatoren umsetzt, wobei R¹ für gesättigte, geradkettige Alkylgruppen mit C₁ bis C₆ und R² für gesättigte, geradkettige oder verzweigte Alkylgruppen mit C₃ bis C₆ oder für gesättigte, geradkettige oder verzweigte Alkylethoxygruppen -CH₂-CH₂-OR³ steht und dabei R³ gesättigte, geradkettige oder gegebenenfalls verzweigte Alkylgruppen mit C₁ bis C₆ umfasst.

Die erfindungsgemäß herstellbaren 2-(N,N-Dialkylamino)-ethoxyverbindungen können als Mikrostrukturregler in Polymerisationsprozessen eingesetzt werden.

## Beschreibung

Die Erfindung umfasst ein Verfahren zur Herstellung von 2-(N,N-Dialkylamino)-ethoxyverbindungen durch Umsetzung von N,N-Dialkylaminoethanol mit Alkyl- bzw. Alkoxyethylchloriden. Die hier beschriebenen 2-(N,N-Dialkylamino)-ethoxyverbindungen können als Mikrostrukturregler in Polymerisationsprozessen eingesetzt werden , wie z. B. der Herstellung von Styrol-Butadien-Kautschuken durch Lösungspolymerisation.

In der Literatur sind mehrere Verfahren zur Alkylierung von Dimethylaminoethanol (= DMAE) beschrieben.

In Org. Mass. Spec. 1979, 14, 1, 31-35 wird die Umsetzung von Alkoholaten mit 2-(N,N-Dimethylamino)-ethylchlorid ohne nähere Angabe von Verfahrensbedingungen beschrieben. Es wurden dort verschiedene 1-Alkoxy-2-dimethylaminoethane synthetisiert, u. a. 1-Methoxy-, 1-Ethoxy-, 1-n-Butyloxy-und 1-i-Propoxy-2-dimethylaminoethan, aber lediglich deren Siedepunkte angegeben.

Die Nachteile dieses Verfahrens, dessen Bedingungen J.Am.Chem. Soc. 1952, 74, 1313- 1315 beschreibt, sind insbesondere darin zu sehen, dass metallisches Natrium zur Alkoholatbildung eingesetzt wird, das technisch schwer zu handhaben ist und gleichzeitig Wasserstoff entsteht, der als leicht brennbares und explosives Gas in einem technischen Verfahren ebenfalls nicht erwünscht ist. Außerdem wird 2-Dimethylaminoethylchloridhydrochlorid eingesetzt und dies führt zu einem zusätzlichen Salzanfall und damit zu größerem Aufwand durch zusätzliche Handhabung von Feststoffen, da das Hydrochlorid anschließend wieder mit Basenüberschuss neutralisiert werden muss. Gleichzeitig wird dadurch der Basenverbrauch entsprechend erhöht, was weder unter wirtschaftlichen noch unter ökologischen Gesichtspunkten wünschenswert ist.

Darüber hinaus ist die sehr lange Reaktionszeit von 24 Stunden für ein industrielles Verfahren sehr ungünstig, wegen der daraus resultierenden niedrigen Raum-Zeit-Ausbeute.

Ein weiterer Nachteil ist der zusätzlicher Einsatz von Lösungsmitteln wie z. B. des hochsiedenden und relativ schwer abtrennbaren Xylols oder von im Überschuss eingesetztem Alkohol, der gleichzeitig als Lösungsmittel dient, sowie die anschließende Extraktion des Produktes mit Ether, wie es für ein Laborverfahren typisch ist.

In J. of the Am. Oil Chem. Soc. 1985, 62, 3, 555-557 wird die Umsetzung von Dimethylaminoethanol und n-Alkylchloriden (C₄, C₆, C₈, C₁₀, C₁₂, C₁₄, C₁₆, C₁₈) in Gegenwart von KOH (fest) beschrieben. Dimethylaminoethanol wird dabei in einem unvorteilhaft hohen Überschuß eingesetzt. So werden bezogen auf 1 Mol-Äquivalent Alkylchlorid insgesamt 4 Mol-Äquivalente Dimethylaminoethanol eingesetzt bei gleichzeitiger Anwesenheit von 1,25 Mol-Äquivalent KOH. Es werden zwar Ausbeuten von 75-83 % erreicht, aber es sind dazu sehr lange Reaktionszeiten von 72 Stunden nötig. Gleichzeitig tritt die Bildung von quartemären Ammoniumsalzen als unerwünschte Nebenprodukte auf, welche nur schwer abtrennbar sind. Über die Umsetzung in einem zweiphasigen System in Gegenwart von Phasentransferkatalysatoren hingegen wird ausgesagt, dass diese zu schlechten Ausbeuten führt (Seite 556 rechte Spalte). Bedingt durch den hohen Überschuss an Dimethylaminoethanol ist darüber hinaus bei der Synthese von kurzkettigen 1-Alkoxy-2-dimethylaminoethanen (Alkoxy= Propyloxy, iso-Butyloxy, n-Butyloxy) die destillative Trennung von DMAE und Produkt aufgrund der nahe beieinanderliegenden Siedepunkte sehr schwierig und dürfte zu weiteren erheblichen Ausbeuteverlusten insbesondere in einem technischen Verfahren führen.

In US 4588843 wird die Umsetzung von DMAE mit Alkylhalogeniden in Gegenwart von Natrium- oder Kaliumhydroxid beschrieben. Dabei werden erhebliche Überschüsse der Reaktanden in für ein industrielles Verfahren unerwünschten Höhe eingesetzt, wie z. B. 10 Mol-Äquivalente DMAE und 4 Mol-Äquivalente KOH bezogen auf 1 Mol-Äquivalent Alkylbromid im einzigen offenbarten Beispiel.

Die Nachteile dieses Verfahrens sind einerseits in den hohen Überschüssen und der damit einhergehenden wässrigen Aufarbeitung zu sehen, die eine Rückführung der im Überschuss eingesetzten Komponenten praktisch verhindert sowie eine entsprechend hohe Abwassermenge von ca. 54 kg/kg Produkt, einschließlich von nichtumgesetzten DMAE, erwarten lässt. Andererseits ist die offenbarte Extraktion mit leichtentzündlichen organischen Lösungsmitteln, wie Ether, für ein technisches Verfahren nicht erwünscht. Des weiteren liegt die im Beispiel offenbarte Ausbeute von nur 37 % sehr niedrig.

In EP 518013 wird die Umsetzung von Dimethylaminoethanol mit Alkylchloriden in Gegenwart von Alkoholaten beschrieben. Entscheidend ist hier die quantitative Überführung von DMAE in sein Alkalialkoholat, was durch Umsetzung mit kurzkettigen Alkalialkoholaten z. B. Natriummethylat erreicht wird. Um diese quantitative Umsetzung zu erreichen, wird der gebildete Alkohol z. B. Methanol durch Zusatz eines Schleppmittels wie beispielsweise Cyclohexan, Toluol oder t-Butylmethylether oder des Reaktionsproduktes 1-Alkoxy-2-dialkylaminoethan selbst azeotrop entfernt. Dabei dient das zugesetzte Schleppmittel insbesondere aber das Produkt 1-Alkoxy-2-dialkylaminoethan selbst als Lösungsmittel, in der Hauptsache, um das Alkalialkoholat des DMAE in Lösung zu halten.

Die Isolierausbeuten dieses Verfahrens nach einmaliger Rektifikation sind mit 82,9 - 91,9 % der Theorie zwar relativ gut und können durch Recyclierung noch weiter gesteigert werden, dennoch gibt es hier mehrere Nachteile.

Durch die Rückführung von großen Produktanteilen von mehr als der vierfachen bis zur etwa fünffachen Menge bezogen auf neu eingesetztes Ausgangsmaterial, wird die Raum-Zeit-Ausbeute entsprechend reduziert, sowie das Produkt überproportional lang thermisch belastet, was zu verstärkter Nebenproduktbildung bzw. unerwünschten Verfärbungen führen kann.

Das verwendete Alkalialkoholat ist vergleichsweise schwierig zu handhaben, da die Alkoholatlösung zunächst zur Trockene eingeengt und das ganze Verfahren unter Luft- und Feuchtigkeitsausschluss durchgeführt werden muss.

Zum anderen wird Dialkylaminoethanol im Überschuss eingesetzt, was die anschließende destillative Aufreinigung erschwert. Außerdem ist eine zweite Destillation unter Zugabe von Alkoholat notwendig, um überschüssiges Dialkylaminoethanol aus dem Produkt zu entfernen und zu einem Reinprodukt zu gelangen, was zu einer weiteren Erhöhung des Aufwandes führt.

Bei Verwendung von kurzkettigen Alkylchloriden wie z. B. Ethylchlorid oder Propylchlorid ist zudem eine Fahrweise unter Druck nötig, um die notwendigen Reaktionstemperaturen von 100 - 115 °C überhaupt zu erreichen.

Des weiteren resultieren lange Reaktionszeiten, wenn man die Alkoholat-Herstellung und die zweifache Destillation mit berücksichtigt und ein insgesamt aufwändiges Verfahren.

Aufgabe der vorliegenden Erfindung war es, die Nachteile der bisherigen Verfahren zu vermeiden oder zu verringern und dabei ein einfaches, praktikables Verfahren zur O-selektiven Alkylierung eines entsprechenden 2-N,N-Dialkylaminoethanols zur Verfügung zu stellen, das ausgehend von preisgünstigen und technisch verfügbaren Rohstoffen wie Alkylchloriden oder Alkoxyalkylchloriden mit möglichst niedrigen Überschüssen bzw. Verbräuchen an Reaktanden bei gleichzeitig möglichst kurzen Reaktionszeiten und hohen Raum-Zeit-Ausbeuten auch im halbtechnischen und technischen Maßstab in gängigen technischen Anlagen durchführbar sein sollte und dabei eine möglichst einfache Form der Aufreinigung, insbesondere in Form einer Destillation, sowie hohe Produktreinheiten ermöglicht.

Gelöst werden diese sowie weitere nicht explizit genannten Aufgaben, die jedoch aus den hierin diskutierten Zusammenhängen ohne weiteres ableitbar oder erschließbar sind durch ein Verfahren nach Anspruch 1. Zweckmäßige Ausformungen und Abwandlungen des erfindungsgemäßen Verfahrens werden in den auf Anspruch 1 rückbezogenen Unteransprüchen unter Schutz gestellt.

Dadurch dass man zur Herstellung von 2-(N,N-Dialkylamino)-ethoxyverbindungen der Formel I, 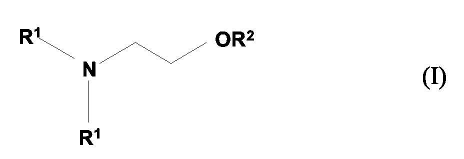 ein Verfahren anwendet, bei dem 2- N,N-Dialkylaminoethanol (= DAAE) der Formel II 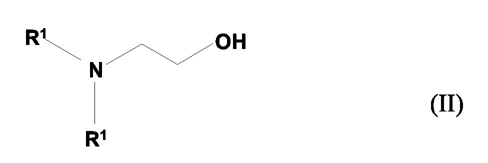 mit Alkylchloriden oder Alkoxyethylchloriden der Formel III

**CI-R**^{**2**} (III)

in Gegenwart von wässrigen Alkalimetallhydroxidlösungen und Phasentransferkatalysatoren umgesetzt werden, wobei R¹ für gesättigte, geradkettige Alkylgruppen mit C₁ bis C₆ und R² für gesättigte, geradkettige oder verzweigte Alkylgruppen mit C₃ bis C₆ oder für gesättigte, geradkettige oder verzweigte Alkylethoxygruppen -CH₂-CH₂-OR³ steht und dabei R³ gesättigte, geradkettige oder ggf. verzweigte Alkylgruppen mit C₁ bis C₆ umfasst, gelingt es, die oben näher bezeichneten Nachteile des Standes der Technik zu überwinden.

Das erfindungsgemäße Verfahren erlaubt die direkte O-selektive Alkylierung von 2-N,N-Dialkylaminoethanol und vermeidet damit den Einsatz von Hydrochloriden und den damit einhergehenden erhöhten Verbrauch von Base zur Neutralisation ebenso wie die erhöhten Salzfrachten der betreffenden Verfahren herkömmlicher Art.

Bei dem erfindungsgemäßen Verfahren können des weiteren preisgünstige und technisch verfügbare Rohstoffe wie Alkylchloride oder Alkoxyethylchloride in vorteilhafter Weise eingesetzt werden. Alternativ können auch die entsprechenden Alkylbromide oder Alkoxyethylbromide eingesetzt werden, sofern diese verfügbar sind.

Außerdem ermöglicht das erfindungsgemäße Verfahren die Verwendung vergleichsweise niedriger Überschüsse bzw. Verbräuche an Reaktanden.

Die Reaktionszeiten des vorliegenden Verfahrens sind mit 1 bis 10 Stunden, insbesondere 3 - 6 Stunden vergleichsweise kurz und tragen auf diese Weise zu hohen Raum-Zeit-Ausbeuten bei.

Das Verfahren ist sowohl im halbtechnischen als auch in gängigen technischen Anlagen durchführbar und zeichnet sich durch eine sehr einfache Form der Aufreinigung aus, die insbesondere mittels einer Destillation von statten gehen kann.

Trotz der Einfachheit werden beim erfindungsgemäße Verfahren gleichzeitig gute Ausbeuten und hohe Produktreinheiten erhalten.
Als Alkylierungsmittel können z. B. Alkylchloride wie 1-Propylchlorid, Isopropylchlorid, 1-Butylchlorid, 2-Butylchlorid, Isobutylchlorid, 1-Pentylchlorid, 1-Hexylchlorid, Neopentylchlorid oder Alkoxyethylchloride wie 2-Ethoxyethylchlorid eingesetzt werden.

Bei der Verwendung von preiswerten Alkylchloriden ist ein Überschuss an Alkylchlorid sinnvoll, um vollständige Umsätze und eine gute Phasentrennung in der Aufarbeitung zu erhalten. Das überschüssige Alkylchlorid kann dann beispielsweise in einer Destillation zurückgewonnen und erneut eingesetzt werden. Grundsätzlich können aber auch Alkylchlorid und DAAE in äquimolaren Verhältnissen eingesetzt werden.

Als basische Komponenten können wässrige Alkalimetallhydroxidlösungen umfassend ein oder auch mehrere Alkalimetallhydroxide ausgewählt aus der Gruppe Lithium-, Natrium-, Kalium-, Rubidium- und Cäsiumhydroxid eingesetzt werden. Vorzugsweise werden wässrige Natrium- bzw. Kaliumhydroxid-Lösungen eingesetzt. Die dabei bevorzugten Konzentrationen liegen bei 10-50 %.

Bei Verwendung hochkonzentrierter Natrium- bzw. Kaliumhydroxid-Lösung fällt im Verlauf der Reaktion Natriumchlorid bzw. Kaliumchlorid aus, das z. B. durch Filtration der Reaktionsmischung leicht abgetrennt werden kann, was von besonderem Vorteil ist.

Als Phasentransferkatalysatoren können beispielsweise quarternäre Ammoniumsalze eingesetzt werden. Vorteilhaft ist es, mindestens einen Vertreter ausgewählt aus der Gruppe von Tetrabutylammoniumbromid, Tetrabutylammoniumchlorid, Tetraethylammoniumbromid, Tetraethylammoniumchlorid, Benzyltrimethylammoniumchlorid, Triethylbenzylammoniumchlorid, Methyltributylammoniumchlorid einzusetzen. Ganz besonders bevorzugt ist die Verwendung von Methyltricaprylammoniumchlorid, das beispielsweise unter dem Namen Aliquat 336 (Firma Henkel) im Handel erhältlich ist.

Die Umsetzung kann sowohl bei Normaldruck als auch bei erhöhtem Druck z. B. in einem Autoklaven oder einer technischen Druckapparatur durchgeführt werden. Unter Normaldruck wird die Reaktion vorzugsweise bei erhöhter Temperatur bis zur Siedetemperatur der Mischung durchgeführt. Hier ist jedoch zur Reduzierung der Reaktionsdauer eine Umsetzung in der Siedehitze besonders vorteilhaft. Erfindungsgemäß wird die Reaktion bei Temperaturen von 30 bis 150 °C, bevorzugt bei Temperaturen von 60 bis 120 °C, ganz besonders bevorzugt jedoch bei Temperaturen von 80 bis 100 °C durchgeführt.

Die Reaktionszeit kann nach dem DAAE -Umsatz festgelegt werden, der z. B. durch gaschromatographische Analyse der organischen Phase bestimmt wird. Üblicherweise liegt der DAAE -Gehalt in der organischen Phase nach Abschluss der Reaktion bei < 2 %. Erfindungsgemäß anzuwendende Reaktionszeiten für die Umsetzung sind 1 - 10 Stunden, bevorzugt aber 3 - 6 Stunden.
Beim erfindungsgemäßen Verfahren wird Alkalimetallhydroxid vorzugsweise in einem Verhältnis von 1 bis 8 Mol-Äquivalenten, besonders bevorzugt aber in einem Verhältnis von 2,5 bis 5 Mol-Äquivalenten jeweils bezogen auf ein Mol-Äquivalent 2- N,N-Dialkylaminoethanol eingesetzt.

Das als Alkylierungsmittel verwendete Alkylchlorid oder Alkoxyethylchlorid wird vorzugsweise in einem Verhältnis von 1,0 bis 2,5 Mol-Äquivalenten, besonders bevorzugt aber in einem Verhältnis von 1,2 bis 2,0 Mol-Äquivalenten bezogen auf ein Mol-Äquivalent 2- N,N-Dialkylaminoethanol eingesetzt.

Der Phasentransferkatalysator wird in einem Verhältnis von 1 bis 10 Mol-%, bevorzugt jedoch in einem Verhältnis von 3 bis 5 Mol-% bezogen auf ein Mol-Äquivalent 2- N,N-Dialkylaminoethanol eingesetzt.

Nach Abschluss der Reaktion wird die Reaktionsmischung abgekühlt und evtl. ausgefallenes Alkalichlorid entweder abfiltriert oder durch Zugabe von Wasser in Lösung gebracht. Nach der Phasentrennung kann unter Umständen ein einmaliges Waschen der organischen Phase mit Wasser oder Natriumchlorid-Lösung sinnvoll sein, um Reste an unumgesetztem DAAE vollständig zu entfernen. Die organische Phase kann erfindungsgemäß anschließend direkt in einer abschließenden Destillation eingesetzt werden, was von besonderem Vorteil ist, weil weitere Schritte wie die einer Lösemittelextraktion - wie in bisherigen Verfahren üblich - damit entfallen. So ist es gleichzeitig möglich die aufwändige Lösemittelextraktion und - rückgewinnung einzusparen, als auch die Gefährdung durch die Handhabung leichtbrennbarer und explosibler Lösemittel zu vermeiden. Ebenso ist eine Zurückführung von bereits gebildetem Produkt in die Reaktionsstufe überflüssig.

Die 1-Alkoxy-2-dialkylaminoethane und 1-Alkoxy- 2-(2-Dialkylaminoethoxy)-ethane können auf diese Weise bereits ohne Recyclierung mit guten Ausbeuten von bis ca. 80 %, in hohen Reinheiten von über 99 % und praktisch ohne Verfärbungen auf eine sehr einfach durchführbare Weise im technischen oder halbtechnischen Maßstab erhalten werden.

### Beispiel 1: 1-Propoxy-2-dimethylaminoethan (PDE)

Zu einer Mischung von 194 g (2,2 mol) Dimethylaminoethanol, 342 g n-Propylchlorid (4,4 mol) und 49 g (0,1 mol) Aliquat 336 wurde bei Raumtemperatur 872 g (10,9 mol) 50 %ige Natronlauge gegeben und die Mischung dann zum Rückfluss erwärmt. Nach 4,5 h Reaktionszeit lag das Verhältnis der drei Reaktionspartner in der organischen Phase Propylchlorid : DMAE : PDE lt. GC-Analyse bei 20 : 2 : 78. Die Mischung wurde auf Raumtemperatur abgekühlt, der ausgefallene Feststoff abfiltriert und die Phasen getrennt. Die organische Phase wurde mit gesättigter NaCl-Lösung gewaschen. Anschließend lag das Verhältnis PrCl : DMAE : PDE bei 19 : 0 : 81. Das Rohprodukt wurde an einer 20 cm-Destillationskolonne mit Glasraschig-Ringen destilliert. Es wurden 210 g PDE (Sdp. 57-58°C bei 234 mbar, 73 % Ausbeute) in der Destillation erhalten.

### Beispiel 2: 1-Butoxy-2-dimethylaminoethan (nBDE)

Zu einer Mischung von 267 g (3,0 mol) DMAE, 556 g (6,0 mol) n-Butylchlorid und 63 g (0,14 mol) Aliquat 336 werden bei Raumtemperatur 960 g (12,0 mol) 50 %ige Natronlauge gegeben und die Mischung dann zum Rückfluss erhitzt. Nach 3 h betrug das Verhältnis der Komponenten n-Butylchlorid : DMAE : nBDE in der organischen Phase laut GC-Analyse 28,4 : 0,6 : 71,0. Die Mischung wurde abgekühlt, filtriert und die organische Phase mit gesättigter NaCl-Lösung gewaschen. Nach Abschluss der Destillation der organischen Phase wurden 339 g nBDE (Sdp. 87-88°C bei 77 mbar, 78 % Ausbeute) erhalten.

### Beispiel 3: 2-(2-Dimethylaminoethoxy)-1-ethoxyethan (DEE)

Eine Mischung von 140 g (1,57 mol) DMAE, 171 g (1,57 mol) Ethoxyethylchlorid, 628 g (7,85 mol) 50 %ige Natronlauge und 35 g (0,08 mol) Aliquat 336 wurde zum Rückfluß erhitzt. Nach 3 h Reaktionszeit lag das Verhältnis der Komponenten DMAE : Ethoxyethylchlorid: DEE bei 25 : 6 : 69. Die Mischung wurde abgekühlt, filtriert und die organische Phase direkt in der Destillation eingesetzt . Es wurden 135 g DEE (Sdp. 77°C bei 37 mbar, 53 % Ausbeute) erhalten.

Da die destillative Trennung von DMAE und DEE hier weniger problematisch war, wurde auf einen vollständigen Umsatz verzichtet und das zurückgewonnene DMAE erneut eingesetzt (40 g, 0,45 mol, 75 % Ausbeute bezogen auf verbrauchtes DMAE).

## Patentansprüche

1. Verfahren zur Herstellung von 2-(N,N-Dialkylamino)-ethoxyverbindungen der Formel I, 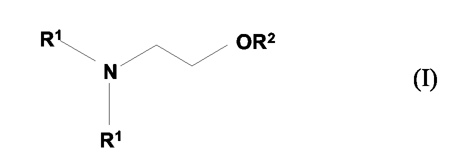 **dadurch gekennzeichnet,**
**dass** man 2- N,N-dialkylaminoethanol der Formel II 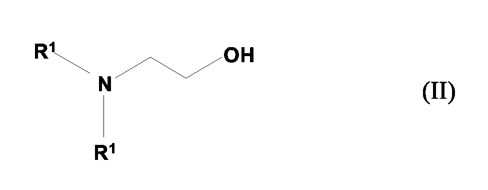 mit Alkylchloriden oder Alkoxyethylchloriden der Formel III
**Cl-R**^{**2**} (III)
in Gegenwart von wässrigen Alkalimetallhydroxidlösungen und Phasentransferkatalysatoren umsetzt, wobei R¹ für gesättigte, geradkettige Alkylgruppen mit C₁ bis C₆ und R² für gesättigte, geradkettige oder verzweigte Alkylgruppen mit C₃ bis C₆ oder für gesättigte, geradkettige oder verzweigte Alkylethoxygruppen -CH₂-CH₂-OR³ steht und dabei R³ gesättigte, geradkettige oder gegebenenfalls verzweigte Alkylgruppen mit C₁ bis C₆ umfasst.

2. Verfahren nach Anspruch 1,
**dadurch gekennzeichnet,**
**dass** man als Alkylchlorid bzw. Alkoxyethylchlorid 1-Propylchlorid, Isopropylchlorid, 1-Butylchlorid, 2-Butylchlorid, Isobutylchlorid, 1-Pentylchlorid, 1-Hexylchlorid, Neopentylchlorid oder 2-Ethoxyethylchlorid einsetzt.

3. Verfahren nach Anspruch 1 oder 2,
**dadurch gekennzeichnet,**
**dass** man wässrigen Alkalimetallhydroxidlösungen mit Konzentrationen von 10 - 50 Gew.% Alkalimetallhydroxid einsetzt.

4. Verfahren nach einem der Ansprüche 1 bis 3,
**dadurch gekennzeichnet,**
**dass** man ein oder mehrere Alkalimetallhydroxide ausgewählt aus der Gruppe Lithium-, Natrium- , Kalium-, Rubidium- und Cäsiumhydroxid einsetzt.

5. Verfahren nach einem der Ansprüche 1 bis 4,
**dadurch gekennzeichnet,**
**dass** man Natrium und /oder Kaliumhydroxid einsetzt.

6. Verfahren nach einem der Ansprüche 1 bis 5,
**dadurch gekennzeichnet,**
**dass** man als Phasentransferkatalysator quarternäre Ammoniumsalze einsetzt.

7. Verfahren nach Anspruch 6,
**dadurch gekennzeichnet,**
**dass** man als Phasentransferkatalysator mindestens einen Vertreter ausgewählt aus der Gruppe von Tetrabutylammoniumbromid, Tetrabutylammoniumchlorid, Tetraethylammoniumbromid, Tetraethylammoniumchlorid, Benzyltrimethylammoniumchlorid, Triethylbenzylammoniumchlorid, Methyltributylammoniumchlorid und Methyltricaprylammoniumchlorid, einsetzt.

8. Verfahren nach einem der Ansprüche 1 bis 7,
**dadurch gekennzeichnet,**
**dass** man die Reaktion bei Temperaturen von 30 bis 150 °C durchführt.

9. Verfahren nach Anspruch 8,
**dadurch gekennzeichnet,**
**dass** man die Reaktion bei Temperaturen von 60 bis 120 °C durchführt.

10. Verfahren nach Anspruch 9,
**dadurch gekennzeichnet,**
**dass** man die Reaktion bei Temperaturen von 80 bis 100 °C durchführt.

11. Verfahren nach einem der Ansprüche 1 bis 10,
**dadurch gekennzeichnet,**
**dass** man eine Reaktionszeit von 1 bis 10 Stunden wählt.

12. Verfahren nach Anspruch 11,
**dadurch gekennzeichnet,**
**dass** man eine Reaktionszeit von 3 bis 6 Stunden wählt.

13. Verfahren nach einem der Ansprüche 1 bis 12,
**dadurch gekennzeichnet,**
**dass** man das Alkalimetallhydroxid in einem Verhältnis von 1 bis 8 Mol-Äquivalente bezogen auf ein Mol-Äquivalent 2- N,N-Dialkylaminoethanol einsetzt.

14. Verfahren nach Anspruch 13,
**dadurch gekennzeichnet,**
**dass** man das Alkalimetallhydroxid in einem Verhältnis von 2,5 bis 5 Mol-Äquivalente bezogen auf ein Mol-Äquivalent 2- N,N-Dialkylaminoethanol einsetzt.

15. Verfahren nach einem der Ansprüche 1 bis 14,
**dadurch gekennzeichnet,**
**dass** man das Alkylchlorid oder das Alkoxyethylchlorid in einem Verhältnis von 1,0 bis 2,5 Mol-Äquivalente bezogen auf ein Mol-Äquivalent 2- N,N-Dialkylaminoethanol einsetzt.

16. Verfahren nach Anspruch 15,
**dadurch gekennzeichnet,**
**dass** man das Alkylchlorid oder das Alkoxyethylchlorid in einem Verhältnis von 1,2 bis 2,0 Mol-Äquivalenten bezogen auf ein Mol-Äquivalent 2- N,N-Dialkylaminoethanol einsetzt.

17. Verfahren nach einem der Ansprüche 1 bis 16,
**dadurch gekennzeichnet,**
**dass** man den Phasentransferkatalysator in einem Verhältnis von 1 bis 10 Mol-% bevorzugt jedoch von 3 bis 5 Mol-% bezogen auf 2- N,N-Dialkylaminoethanol einsetzt.
